# FASCICULE DE BREVET EUROPEEN

(11) **EP 1 885 267 B1**
(45) Date de publication et mention de la délivrance du brevet: **23.04.2014**
(21) Numéro de dépôt: 06764613.3
(22) Date de dépôt: 12.05.2006
(51) Int. Cl.: A61B 17/72, A61B 17/17

(54) **APPAREILLAGE D'ARTHRODESE POUR UNE ARTICULATION, DU GENRE ARTICULATION DE LA CHEVILLE**
ARTHRODESEGERÄT FÜR DAS FUSSGELENK
JOINT FUSION APPARATUS FOR THE ANKLE-TYPE JOINT

(30) Priorité: 13.05.2005 FR 0504851
(43) Date de publication de la demande: 13.02.2008
(73) Titulaire: Newdeal, 69006 Lyon (FR)
(72) Inventeur: BRODSKY, James, White, Dallas, TX 75220 (US); GAUNEAU, Bertrand, Xavier, François, F-69570 DARDILLY (FR)
(74) Mandataire: Martin, Didier Roland Valéry
(86) Numéro de dépôt international: PCT/FR2006/001070
(87) Numéro de publication internationale: WO 2006/120355

(56) Documents cités:
- EP-A- 1 393 696
- WO-A-2004/014243
- US-A1- 2002 151 898
- US-A1- 2003 097 131
- US-B1- 6 197 029

## Description

### DOMAINE TECHNIQUE

La présente invention se rapporte au domaine technique général des appareillages destinés à être utilisés pour réaliser une arthrodèse.

La présente invention se rapporte à un appareillage d'arthrodèse pour une articulation formée par au moins un premier et un deuxième os, du genre articulation de la cheville, ledit appareillage comportant :
- un clou d'arthrodèse, pourvu de moyens de fixation avec les os formant l'articulation,
- un instrument de mise en place dudit clou d'arthrodèse au sein de l'articulation, comportant une base ainsi qu'un moyen de compression, destiné à permettre le rapprochement des premier et deuxième os formant l'articulation,
- un moyen de liaison, servant d'interface entre l'instrument et le clou d'arthrodèse.

### TECHNIQUE ANTERIEURE

Dans une application préférentielle, mais non exclusive, l'appareillage conforme à l'invention sera plus particulièrement destiné et conçu pour réaliser une arthrodèse de l'articulation de la cheville, étant entendu que des applications à d'autres articulations, notamment à celle de l'épaule, sont aussi envisageables.

Une arthrodèse est une intervention chirurgicale destinée à supprimer presque complètement la mobilité d'une articulation en provoquant une *« fusion osseuse* ». Une telle intervention chirurgicale - peut s'avérer nécessaire dans le cas où le patient souffre d'arthrose sévère et finale.

Pour réaliser de telles interventions, il est connu d'utiliser un appareillage d'arthrodèse comportant d'une part un clou d'arthrodèse, destiné à être implanté au sein de l'articulation, et d'autre part un instrument de mise en place du clou d'arthrodèse.

Le clou d'arthrodèse est généralement introduit dans des logements ménagés successivement à travers les différents os formant l'articulation, et il comporte des trous de fixation destinés à coopérer avec des vis de fixation pour le solidariser avec les os formant l'articulation.

Il est connu, afin de mettre en place de tels, clous d'arthrodèse, d'avoir recours à un instrument comportant un moyen de compression, destiné à permettre le rapprochement des os formant l'articulation, et par exemple, dans le cas de l'articulation de la cheville, le rapprochement du calcanéum et du tibia.

L'étape de compression revêt un caractère très important dans l'intervention chirurgicale dans la mesure où cette étape conditionne le positionnement relatif des os formant l'articulation, un mauvais positionnement étant susceptible de générer une gêne pour le patient.

Or, dans le cas de l'articulation de la cheville, le moyen de compression est généralement réalisé à l'aide d'une portée, agencée pour venir en appui contre le talon et susceptible d'être déplacée pour exercer une pression contre ce dernier, rapprochant ainsi le calcanéum du tibia.

La technique opératoire généralement utilisée est la suivante :
- le chirurgien solidarise, à l'aide de vis de fixation, le clou d'arthrodèse avec le tibia,
- le chirurgien déplace ensuite la portée, de telle sorte qu'elle vienne en appui contre le talon et comprime l'articulation, rapprochant ainsi le tibia du calcanéum,
- le chirurgien solidarise enfin le clou avec le calcanéum, tout en maintenant la pression exercée par la portée sur le talon.

Le document US 2003/097131 A1 sur lequel le préambule de la revendication 1 est basé, décrit un appareillage d'arthrodèse pour l'articulation de la cheville, ledit appareillage comportant un clou d'arthrodèse, pourvu de moyens de fixation avec les os formant l'articulation, un instrument de mise en place dudit clou d'arthrodèse au sein de l'articulation, comportant une base ainsi qu'un moyen de compression, destiné à permettre le rapprochement des premier et deuxième os formant l'articulation, et un moyen de liaison, servant d'interface entre l'instrument et le clou d'arthrodèse.

Le document US2002/0151898 A1 décrit un clou intramédullaire pourvu de moyens de fixation et de moyens de guidage.

De tels appareillages d'arthrodèse, s'ils permettent d'obtenir des résultats intéressants en matière d'immobilisation de l'articulation, souffrent néanmoins d'inconvénients non négligeables.

En particulier, il existe un risque, avec les appareillages de l'art antérieur, que lors de l'étape de compression, le clou traverse le cortex plantaire du calcanéum et fasse saillie à l'extérieur du faciès plantaire.

Avec de tels appareillages, le chirurgien doit donc tout particulièrement veiller à ne pas effectuer une compression trop importante de l'articulation. Une telle contrainte constitue un inconvénient non négligeable pour le chirurgien, d'autant plus qu'il est souvent difficile, voire impossible, avec les appareillages de l'art antérieur, de contrôler précisément l'intensité de la compression.

En outre, l'utilisation de tels appareillages peut conduire à un endommagement des tissus mous du faciès plantaire, tissus situés entre la portée de compression et le calcanéum.

En effet, si l'intensité de la compression est trop importante, la pression exercée par la portée peut générer une nécrose de la peau au niveau du talon.

Par ailleurs, le montage des vis de fixation dans le calcanéum et l'astragale étant effectué après l'étape de compression, c'est-à-dire après le déplacement de l'astragale et du calcanéum relativement au tibia, il peut arriver que les vis de fixation soient mal positionnées dans les os, c'est-à-dire qu'elles soient positionnées trop près de la périphérie d'un os, voire positionnées dans la zone de jonction entre deux os. Un clou d'arthrodèse ainsi fixé n'est alors pas en mesure d'assurer un maintien correct de l'articulation.

### EXPOSE DE L'INVENTION

Les objets assignés à l'invention visent en conséquence à porter remède aux différents inconvénients énumérés précédemment et à proposer un nouvel appareillage d'arthrodèse pour une articulation du genre articulation de la cheville, qui permette d'effectuer une compression efficace de l'articulation sans risquer d'endommager d'une part le cortex, et d'autre part les tissus mous voisins des os formant l'articulation.

Un autre objet de l'invention vise à proposer un nouvel appareillage d'arthrodèse présentant une bonne rigidité d'ensemble lors de l'étape de compression.

Un autre objet de l'invention vise à proposer un nouvel appareillage d'arthrodèse permettant d'effectuer une compression contrôlée et précise de l'articulation.

Un autre objet de l'invention vise à proposer un nouvel appareillage d'arthrodèse permettant une fixation du clou d'arthrodèse adaptée à l'anatomie de l'articulation.

Un autre objet de l'invention vise à proposer un nouvel appareillage d'arthrodèse assurant un maintien confortable de l'articulation.

Un autre objet de l'invention vise à proposer un nouvel appareillage d'arthrodèse facilitant le montage des vis de fixation du clou relativement aux os formant l'articulation.

Un autre objet de l'invention vise à proposer un nouvel appareillage d'arthrodèse présentant un caractère modulable en fonction de l'anatomie de l'articulation tout en étant simple à utiliser.

Un autre objet de l'invention vise à proposer un nouvel appareillage d'arthrodèse dont la manipulation est facilitée et qui permet de réduire les erreurs opératoires.

Les objets assignés à l'invention visent également à proposer un clou d'arthrodèse destiné à être utilisé au sein de l'appareillage sus-mentionné. Les objets assignés à l'invention sont atteints à l'aide d'un appareillage d'arthrodèse pour une articulation formée par au moins un premier et deuxième os, du genre articulation de la cheville, ledit appareillage comportant :
- un clou d'arthrodèse pourvu de moyens de fixation avec les os formant l'articulation,
- un instrument de mise en place dudit clou d'arthrodèse au sein de l'articulation, comportant une base ainsi qu'un moyen de compression, destiné à permettre le rapprochement des premier et deuxième os formant l'articulation,
- un moyen de liaison servant d'interface entre l'instrument et le clou d'arthrodèse,
caractérisé en ce que le moyen de compression comporte :
- un moyen de commande agencé de manière à générer, par l'intermédiaire dudit moyen de liaison, un déplacement du clou d'arthrodèse relativement à ladite base selon un axe longitudinal sensiblement parallèle à l'axe d'extension dudit clou,
- un moyen d'immobilisation, agencé pour immobiliser le deuxième os relativement à la base,
ledit clou comportant un moyen de guidage, ledit moyen de guidage et ledit moyen d'immobilisation étant agencés et conformés pour coopérer ensemble de manière à autoriser le déplacement du clou relativement au moyen d'immobilisation, et à permettre, lorsque le clou est solidarisé avec le premier os, le rapprochement des premier et deuxième os.

### DESCRIPTIF SOMMAIRE DES DESSINS

D'autres avantages et objets de l'invention apparaîtront plus en détails à la lecture de la description qui suit, et à l'aide des dessins annexés fournis à titre purement illustratif et non limitatif, dans lesquels :
- La figure 1 illustre, selon une vue de côté, une partie de l'appareillage d'arthrodèse conforme à l'invention dans l'étape de solidarisation du clou d'arthrodèse avec le calcanéum.
- La figure 2 illustre, selon une vue en perspective, deux variantes de réalisation de clous d'arthrodèse conformes à l'invention.
- La figure 3 illustre, selon une vue de face, un appareillage d'arthrodèse conforme à l'invention, dans sa position d'avant la mise en compression.
- La figure 4 illustre, selon une vue de face, un appareillage d'arthrodèse conforme à l'invention dans sa position d'après la mise en compression.
- La figure 5 illustre, selon une vue en perspective, un appareillage d'arthrodèse conforme à l'invention lors de la fixation du clou d'arthrodèse (articulation non représentée).
- La figure 6 illustre, selon une vue en coupe, une variante de réalisation d'un appareillage d'arthrodèse conforme à l'invention, dans sa position d'avant la mise en compression.

### MEILLEURE MANIERE DE REALISER L'INVENTION

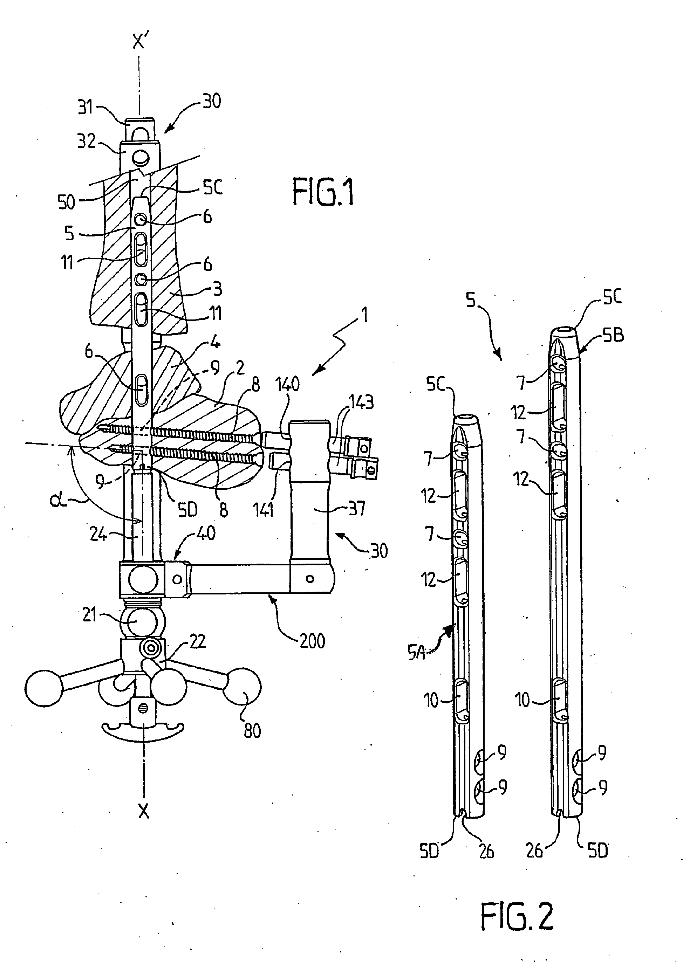

Les figures 1, 3, 4 et 5 illustrent un appareillage 1 d'arthrodèse conforme à l'invention. Un tel appareillage est conçu pour être utilisé dans le cadre d'une arthrodèse, notamment lorsqu'une articulation, et particulièrement l'articulation de la cheville, se trouve dans un état si dégradé que d'autres interventions chirurgicales moins sévères, telles que par exemple celles consistant à poser des prothèses de cheville, s'avéreraient inefficaces. Il devient nécessaire, dans ce cas, d'immobiliser complètement l'articulation.

Au sens de l'invention, l'articulation est formée par au moins deux os, à savoir un premier os 2 et un deuxième os 3, mais peut également comporter un troisième os 4 situé entre le premier os 2 et le deuxième os 3.

L'appareillage 1 d'arthrodèse conforme à l'invention est tout particulièrement adapté à la réparation de l'articulation de la cheville. Toutefois, l'appareillage 1 d'arthrodèse selon l'invention peut également être utilisé pour d'autres opérations d'arthrodèse, tel que l'enclouage centro-médulaire, et ce, sans sortir du cadre de l'invention. Pour ces autres applications, des adaptations devront cependant être prévues, notamment sur le plan dimensionnel.

Dans la suite de la description, on fera référence au premier os 2, au deuxième os 3 et éventuellement au troisième os 4 formant l'articulation, étant entendu que si l'articulation est une articulation de cheville, le premier os 2 correspond au calcanéum, le deuxième os 3 correspond au tibia et le troisième os 4 correspondant à l'astragale.

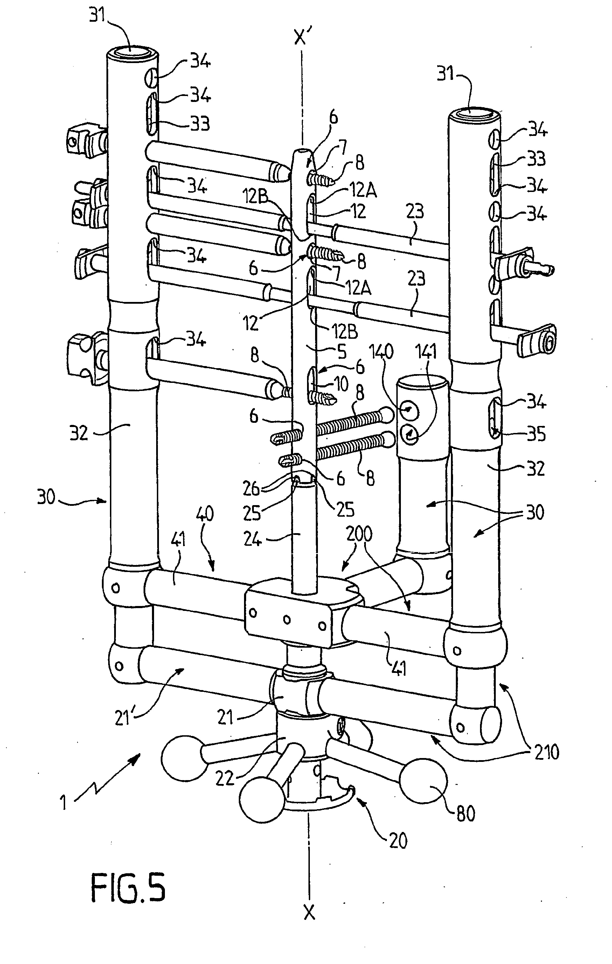

Selon l'invention, l'appareillage 1 d'arthrodèse comporte un clou 5 d'arthrodèse pourvu de moyens de fixation 6 avec les os 2, 3, 4 formant l'articulation (figures 1 et 5).

Tel que cela est représenté sur les figures 1 à 3, le clou 5 se présente avantageusement sous la forme d'un cylindre préférentiellement creux qui s'étend entre deux extrémités 5C, 5D. L'une des extrémités 5C est de préférence légèrement chanfreinée pour faciliter son implantation au sein de l'articulation. Le clou 5 est avantageusement réalisé à partir d'un matériau métallique bio-compatible par nature ou rendu bio-compatible à la suite d'un traitement.

Selon une variante de réalisation préférentielle, le clou 5 est monolithique.

De façon particulièrement avantageuse, le clou 5 d'arthrodèse est monté à l'intérieur d'un logement ménagé au sein de l'articulation, et dont au moins une partie se trouve dans chacun des os 2, 3, 4 formant l'articulation, de telle sorte que le clou 5 s'étende selon un axe longitudinal X-X' parallèle à l'axe d'extension du deuxième os 3, par exemple le tibia.

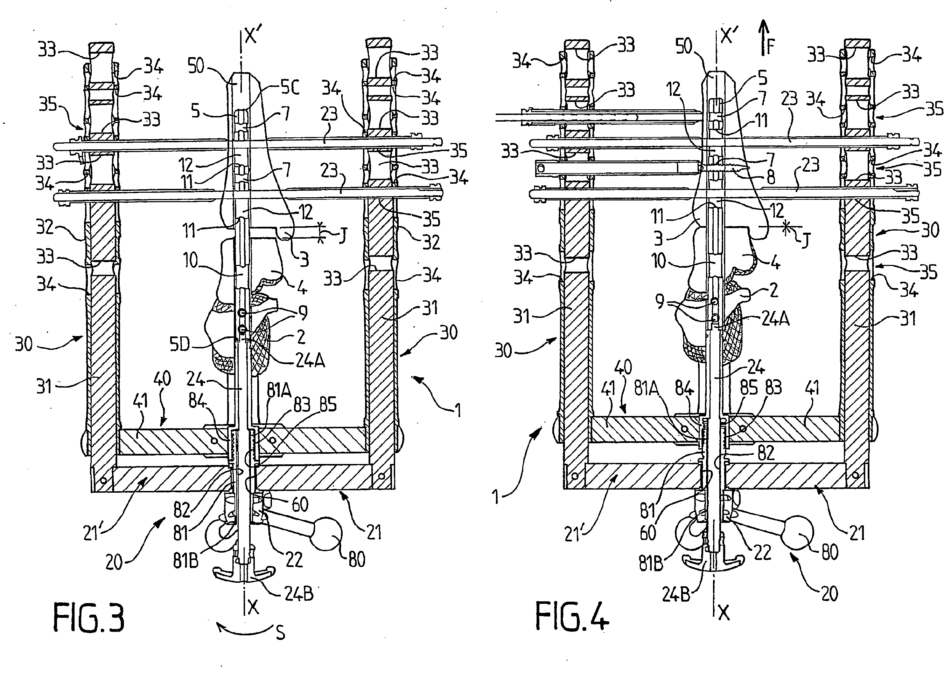

Avantageusement, les moyens de fixation 6 comportent deux trous de fixation 7 (figures 3, 4 et 5). Chacun des trous de fixation 7 est avantageusement destiné à coopérer avec au moins une vis de fixation 8 associée de manière à solidariser le clou 5 avec le deuxième os 3 (figure 4). Bien évidemment, les moyens de fixation 6 peuvent comporter un seul trou de fixation 7, mais la configuration à deux trous de fixation 7 est préférée car elle garantit *a priori* un meilleur maintien du clou au sein de l'articulation.

Les moyens de fixation 6 du clou 5 comportent de préférence au moins un orifice de fixation 9 destiné à coopérer avec au moins une vis de fixation 8 associée, de manière à solidariser le clou 5 avec le premier os 2 (figures 1, 3 et 4). Les vis de fixation 8 utilisées sont celles classiquement utilisées dans le domaine et sont bien connues de l'homme du métier.

De façon préférentielle, l'orifice de fixation 9 s'étend en oblique relativement à l'axe longitudinal X-X', c'est-à-dire qu'il forme un conduit, de préférence sensiblement cylindrique, dont l'axe principal d'extension est oblique par rapport à l'axe longitudinal X-X'. Cette obliquité est notamment destinée à améliorer la fixation du clou dans le premier os 2, par exemple le calcanéum, en tenant compte de l'anatomie particulière de ce dernier.

De façon particulièrement avantageuse, l'orifice de fixation 9 oblique s'étend selon une direction d'extension Y-Y' formant un angle α compris entre 95° et 100°, avec l'axe longitudinal X-X' (figure 1).

De façon encore plus avantageuse, et tel que cela est représenté sur la figure 1, les moyens de fixation 6 comportent deux orifices de fixation 9 du premier os 2. Ces deux orifices de fixation 9 s'étendent de préférence en oblique relativement à l'axe longitudinal X-X'.

Avantageusement, et tel que cela est représenté sur les figures 3, 4 et 5, les moyens de fixation 6 comportent une ouverture de fixation 10 agencée pour recevoir une vis de fixation associée lorsque l'articulation comporte un troisième os 4, par exemple l'astragale, situé entre le premier os 2 et le deuxième os 3, de manière à solidariser sensiblement le clou 5 avec le troisième os 4.

Bien évidemment, dans les cas où l'articulation ne comporte pas de troisième os 4, ou encore dans les cas où l'articulation comporte bien un troisième os 4 mais que ce dernier est fortement dégradé, une telle ouverture de fixation 10 n'est pas nécessaire.

De manière particulièrement avantageuse, l'ouverture de fixation 10 est de préférence oblongue, c'est-à-dire qu'elle est préférentiellement allongée dans la direction parallèle à l'axe longitudinal X-X'. Une telle forme de l'ouverture de fixation 10 est préférée à la forme circulaire afin d'autoriser une certaine mobilité axiale du troisième os 4 dans la direction de l'axe longitudinal X-X', de manière à améliorer sensiblement le confort du patient.

Selon l'invention, l'appareillage 1 d'arthrodèse comporte un instrument 20 de mise en place du clou 5 d'arthrodèse au sein de l'articulation.

L'instrument 20 comporte une base. 21 fixe lors de l'opération chirurgicale, ainsi qu'un moyen de compression 22, 23 destiné à permettre le rapprochement des premier os 2 et deuxième os 3 formant l'articulation.

Selon l'invention, l'appareillage 1 d'arthrodèse comprend également un moyen de liaison 24 servant d'interface entre l'instrument 20 et le clou 5 d'arthrodèse.

Le moyen de liaison 24 peut avantageusement se présenter sous la forme d'une tige, apte à se déplacer longitudinalement le long de l'axe longitudinal X-X'. En particulier, le moyen de liaison 24 et la base 21 sont de préférence agencés de telle manière que le moyen de liaison 24 puisse coulisser à l'intérieur de ladite base 21 (figures 3 et 4).

A cet effet, la base 21 est préférentiellement formée par une platine de compression 210, de préférence fixe et métallique, au sein de laquelle est ménagé un alésage 60 permettant le passage de la tige 24 (figures 3 et 4).

Le clou 5 d'arthrodèse repose préférentiellement sur le moyen de liaison 24 de telle sorte que le déplacement du moyen de liaison 24 entraîne un déplacement sensiblement identique du clou 5.

Le moyen de liaison 24 s'étend avantageusement entre deux extrémités 24A, 24B (figures 3 et 4) et l'extrémité 24A se présente de préférence sous la forme d'un embout fileté, sur lequel l'extrémité 5D correspondante du clou 5 est destinée à être enfilée et vissée.

Afin de faciliter le positionnement du clou 5 relativement au moyen de liaison 24, et d'éviter au chirurgien de se tromper dans l'orientation du clou 5, le moyen de liaison 24 et le clou 5 comportent avantageusement des moyens de repérage 25, 26.

Tel que cela est représenté sur la figure 5, les moyens de repérage 25, 26 sont de préférence formés par trois ergots 25 et trois fentes 26 agencés de telle manière que chaque fente 26 ne puisse être associée qu'avec un seul ergot 25 correspondant.

Tel que cela est représenté sur la figure 2, les fentes 26 sont de préférence ménagées sur l'extrémité 5D du clou 5 destinée à venir en contact avec l'extrémité 24A correspondante du moyen de liaison 24 dans laquelle sont ménagés les ergots 25.

Bien évidemment, il est également envisageable de pourvoir le clou 5 d'ergots destinés à coopérer avec des fentes correspondantes ménagées sur le moyen de liaison 24.

Selon une variante de réalisation préférentielle de l'invention, les moyens de repérage 25, 26 comportent donc trois paires formées chacune par un ergot 25 et une fente 26, lesdites paires étant situées sur le pourtour de la jonction entre le clou 5 et le moyen de liaison 24, et orientées sensiblement à 90 degrés les unes par rapport aux autres.

Une telle configuration des moyens de repérage 25, 26 permet notamment d'éviter que le chirurgien ne monte le clou 5 dans une autre orientation que celle prévue, ce qui provoquerait une mauvaise orientation des moyens de fixation 6 du clou 5 relativement aux os 2, 3, 4 formant l'articulation.

Tel que cela est représenté sur la figure 5, le clou 5 est ainsi avantageusement enfilé sur le moyen de liaison 24 par l'une de ses extrémités 5D pour venir occuper une position stable et fixe d'insertion.

Selon l'invention, le moyen de compression 22, 23 comporte un moyen de commande 22 agencé de manière à générer, par l'intermédiaire du moyen de liaison 24, un déplacement du clou 5 d'arthrodèse relativement à la base 21.

De façon préférentielle, le moyen de commande 22 se présente sous la forme d'un volant 80 (ou d'une molette), monté mobile en rotation autour de l'axe longitudinal X-X' sur la base 21 (ou sur la platine de compression 210) par l'intermédiaire d'une tige creuse 81 (figures 3 et 4).

La tige creuse 81 est montée avec une possibilité de rotation autour de l'axe longitudinal X-X', au sein de l'alésage 60.

La tige creuse 81 comporte un passage intérieur 82 au sein duquel le moyen de liaison 24 est susceptible de coulisser selon l'axe longitudinal X-X'.

La tige creuse 81 s'étend entre une première extrémité 81A, pourvue d'un filetage externe 83, et une deuxième extrémité 81 B, solidarisée avec le volant 80, de telle sorte que la rotation du volant 80 autour de l'axe longitudinal X-X' entraîne la rotation identique de la tige creuse 81.

Tel que cela est illustré sur les figures 3 et 4, le moyen de liaison 24 est fixé à un embout creux 84, comportant un filetage interne 85, apte à coopérer avec le filetage externe 83 de la tige creuse 81, de telle sorte que la rotation du volant 80 suivant le sens de rotation S entraîne la rotation de la tige creuse 81 et, par coopération des filetages interne et externe 85, 83, la translation de l'embout creux 84, du moyen de liaison 24 et du clou 5 associés, selon une direction de compression F (figure 4) parallèle à l'axe longitudinal X-X'.

Ainsi, la mise en rotation du moyen de commande 22 relativement à la base 21 provoque la translation du moyen de liaison 24 et du clou 5 associés selon l'axe longitudinal X-X'.

Il est bien envisageable, sans sortir du cadre de l'invention, de remplacer le volant 80 par tout autre moyen de commande approprié, par exemple par un pistolet de compression du type à cliquet (non représenté), qui possède notamment l'intérêt de permettre un bon contrôle de l'intensité de la compression réalisée.

Selon l'invention, le moyen de compression 22, 23 comporte également un moyen d'immobilisation 23 agencé pour immobiliser le deuxième os 3, par exemple le tibia, relativement à la base 21 et plus précisément à la platine de compression 210 (figure 1).

Une telle immobilisation du deuxième os 3 est préférable afin d'éviter que le déplacement du clou 5 n'entraîne le déplacement du deuxième os 3.

Bien évidemment, le déplacement du clou 5 relativement au deuxième os 3 n'est possible que si ce dernier n'a pas été solidarisé avec le clou 5 à l'aide des vis de fixation 8, ce qui implique que l'étape de compression, dans laquelle on effectue un déplacement du clou 5 d'arthrodèse au sein de l'articulation, doit être réalisée avant l'étape de fixation du clou 5 relativement au deuxième os 3.

Selon l'invention, le clou 5 comporte un moyen de guidage 11, ledit moyen de guidage 11 et ledit moyen d'immobilisation 23 étant agencés et conformés pour coopérer ensemble de manière à autoriser le déplacement du clou 5 relativement au moyen d'immobilisation 23 et à permettre, lorsque le clou 5 est solidarisé avec le premier os 2 le rapprochement des premier os 2 et deuxième os 3.

Ainsi, afin de permettre le rapprochement des premier os 2 et deuxième os 3, il est préférable de solidariser au préalable le clou 5 avec le premier os 2 à l'aide des moyens de fixation 6, en faisant coopérer par exemple le ou les orifices de fixation 9 avec des vis de fixation 8 (figure 1).

Ainsi, lorsque le clou 5 est solidarisé avec le premier os 2, l'actionnement du moyen de commande 22 permet d'assurer le déplacement du clou 5 et du premier os 2 associé, rapprochant ainsi le premier os 2 du deuxième os 3. Grâce à ce montage, et en particulier grâce à la coopération du moyen de guidage 11 avec le moyen d'immobilisation 23, on peut effectuer une compression efficace de l'articulation, tout en contrôlant, grâce à l'agencement particulier du moyen de guidage 11, l'amplitude de déplacement du clou 5 et donc l'intensité de la compression.

En outre, le premier os 2 étant solidarisé et se déplaçant avec le clou 5 lors de la compression, l'utilisation de l'appareillage 1 permet de réduire voire même d'éliminer complètement le risque de perforer le cortex du premier os 2 avec le clou 5 lors de la compression.

Avantageusement, et tel que cela est représenté sur les figures 3 et 4, le moyen de guidage 11 est formé par au moins une cavité 12 oblongue qui présente une forme sensiblement allongée selon l'axe longitudinal X-X'. La cavité 12 oblongue est préférentiellement ménagée dans l'épaisseur du clou 5 de manière à s'étendre selon un axe sécant de l'axe longitudinal X-X'. Ainsi, la cavité 12 oblongue pourra par exemple s'étendre en profondeur selon un axe perpendiculaire de l'axe longitudinal X-X'.

Selon une variante préférentielle de l'invention, la cavité 12 oblongue est traversante, c'est-à-dire qu'elle débouche de part et d'autre du clou 5 d'arthrodèse.

La cavité 12 oblongue s'étend de préférence sur une longueur d'environ 12 mm le long de l'axe longitudinal X-X' de manière à autoriser un déplacement sensiblement équivalent du clou 5 lors de la compression. Les extrémités 12A, 12B de la cavité 12, alignées sur l'axe longitudinal X-X', forment avantageusement des moyens de butée à l'encontre du moyen d'immobilisation 23 (figure 5).

De façon encore plus préférentielle, le moyen de guidage 11 est formé par au moins deux cavités 12 oblongues ménagées l'une au dessus de l'autre le long du clou 5 et en alignement l'une de l'autre selon l'axe longitudinal X-X' (figures 3 et 4).

Avantageusement, les trous de fixation 7, les cavités 12 et l'ouverture de fixation 10 traversent le clou 5 selon un axe sensiblement perpendiculaire à l'axe d'extension des orifices de fixation 9 de manière à permettre une fixation par abord latéral du deuxième os 3 et du troisième os 4, et au contraire une fixation par abord antérieur-postérieur du premier os 2.

Avantageusement, le moyen d'immobilisation du deuxième os 3 est formé par au moins une tige d'immobilisation 23 conformée de manière à traverser, au moins en partie, d'une part le deuxième os 3 pour le maintenir immobile relativement à la base 21 et d'autre part la ou les cavités 12 oblongues (figures 3 et 4).

Grâce à l'agencement particulier de la tige d'immobilisation 23 et de la cavité 12 oblongue, le clou 5 peut se déplacer relativement à la tige d'immobilisation 23 sur une longueur maximale correspondant sensiblement à la longueur de la cavité 12 oblongue, soit de préférence une longueur d'environ 12 mm.

De façon encore plus avantageuse, le moyen d'immobilisation est formé par deux tiges d'immobilisation 23. Une telle mesure a pour effet d'améliorer la rigidité globale de l'appareillage 1 d'arthrodèse lors de la mise en compression de l'articulation.

Chacune des tiges d'immobilisation 23 coopère alors avec une cavité 12 oblongue associée.

Avantageusement, et tel que cela est représenté sur les figures, l'instrument 20 comporte des moyens de visée 30 agencés de manière à faciliter le repérage du positionnement des moyens de fixation 6 sur le clou 5.

En particulier, les moyens de visée 30 sont préférentiellement agencés de manière à permettre le montage d'une part des vis de fixation 8 et leur guidage vers les moyens de fixation 6 ménagés sur le clou 5, et d'autre part des tiges d'immobilisation 23.

De façon particulièrement avantageuse, les moyens de visée 30 comportent au moins un bras 31 monté sur la base 21 de manière à s'étendre sensiblement parallèlement à l'axe longitudinal X-X'.

Le bras 31 est de préférence monté de façon solidaire sur la base 21 de manière à rester immobile relativement à ladite base 21 lors de l'étape de compression et en particulier lorsque le clou 5 est déplacé le long de l'axe longitudinal X-X'.

De façon particulièrement avantageuse, les moyens de visée 30 comportent deux bras 31 (figures 3 et 4). Les deux bras 31 sont de préférence disposés de part et d'autre du clou 5 positionné sur l'instrument 20 et en regard des cavités 12 oblongues. Les deux bras 31 sont ainsi préférentiellement disposés latéralement relativement à l'articulation, de manière à permettre la fixation des vis de fixation 8 par abord latéral (figure 4).

L'utilisation de deux bras 31 permet ainsi d'améliorer sensiblement la rigidité globale de l'instrument 20 lors de l'étape de compression, cette configuration permettant notamment d'obtenir des taux de compression élevés et ce sans générer de déflection indésirable du clou 5.

Selon la variante préférentielle de réalisation de l'invention illustrée sur les figures 3 et 4, le ou les bras sont fixés à la base 21 et s'étendent perpendiculairement à cette dernière de manière à former, avec ladite base 21, la platine de compression 210. De façon préférentielle, la base 21 et les bras 31 sont en métal, et la platine de compression 210 forme un ensemble métallique monobloc dissocié du moyen de liaison 24 et mobile vis-à-vis de ce dernier.

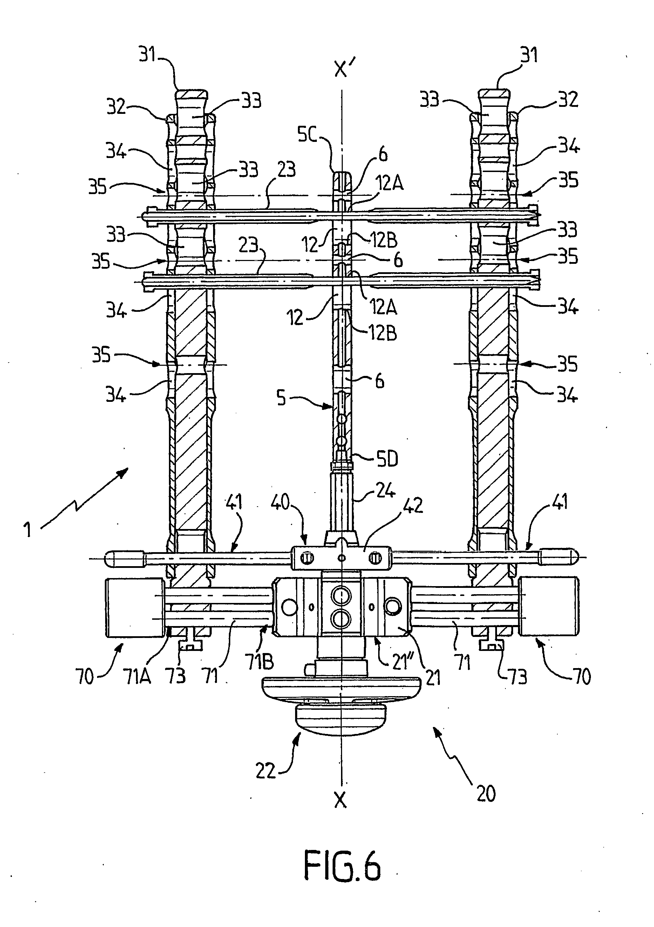

Selon une variante de réalisation de l'invention illustrée sur la figure 6, le bras 31 est monté sur la base 21 de manière amovible, par l'intermédiaire d'une patte 70. Selon cette variante, la base 21 se présente sous la forme d'un noyau central 21". La patte 70 comporte au moins une barrette 71, préférentiellement formée par un tube cylindrique s'étendant entre deux extrémités 71A, 71 B, lequel traverse le bras 31 et est solidarisé avec ce dernier vers une de ses extrémités 71A à l'aide d'un bouton de serrage 73.

L'autre extrémité 71 B de la barrette 71 est avantageusement montée sur la base 21. A cet effet, la base 21 comporte au moins un évidemment au sein duquel l'une des extrémités 71 B de la barrette 71 peut être introduite.

De façon encore plus préférentielle, la patte 70 comporte deux barrettes 71 et la base 21 comporte deux évidements associés.

Un tel montage permet notamment de régler l'écartement entre le bras 31 et le clou 5, par translation du bras 31 le long des barrettes 71, et notamment de rapprocher le bras 31 du clou 5 afin d'améliorer la rigidité de l'appareillage lors de l'étape de compression.

Avantageusement, les moyens de visée 30 comportent au moins un manchon 32 monté mobile sur au moins l'un des bras 31 avec une possibilité de coulissement selon l'axe longitudinal X-X' (figures 3 et 4). Ainsi, la figure 3 montre l'appareillage 1 d'arthrodèse dans lequel le manchon 32 présente une position précédant la mise en compression, tandis que la figure 4 illustre ce même manchon 32 dans une position suivant la mise en compression, c'est-à-dire après déplacement du manchon 32 le long de l'axe longitudinal X-X'.

De façon particulièrement avantageuse, et tel que cela est représenté sur les figures 3 et 4, le bras 31 est pourvu de passages 33 tandis que le manchon 32 est pourvu de trous de visée 34. Les trous de visée 34 et les passages 33 sont agencés de telle manière que par déplacement du manchon 32, au moins une partie des trous de visée 34 puisse être mise en regard d'une partie des passages 33, de manière à former ainsi des lumières de visée 35 situées chacune en vis-à-vis soit d'un moyen de fixation 6, soit d'une cavité 12 oblongue (figure 4).

De telles lumières de visée 35 permettent ainsi le montage respectivement d'une vis de fixation 8 ou d'une tige d'immobilisation 23 sur le clou 5.

Avantageusement, l'instrument 20 comporte un moyen de maintien 40 agencé pour solidariser le manchon 32 avec le moyen de liaison 24, et donc le clou 5 de telle sorte que le déplacement du clou 5 selon l'axe longitudinal X-X' entraîne un déplacement sensiblement identique du manchon 32 (figures 3 à 6).

Avantageusement, et tel que cela est illustré sur les figures 3 et 4, le moyen de maintien 40 est solidarisé avec le moyen de liaison 24 par l'intermédiaire de l'embout creux 84.

Le moyen de maintien 40 comprend également au moins une branche 41 solidarisée d'une part, vers une de ses extrémités, avec le moyen de liaison 24, notamment avec l'embout creux 84, et d'autre part, vers l'autre extrémité, avec le manchon 32, de telle sorte que le déplacement de moyen de liaison 24 entraîne un déplacement sensiblement identique du manchon 32. L'instrument 20 comporte de préférence deux bras 31 et deux manchons 32 associés, et le moyen de maintien 40 comporte de préférence deux branches 41 associées respectivement à chacun des manchons 32.

De façon préférentielle, et tel que cela est illustré sur la figure 5, le moyen de maintien 40 et les manchons 32 forment un ensemble monobloc, apte à se déplacer de façon unitaire.

De façon particulièrement avantageuse, certains des passages 33 ménagés dans le bras 31 sont agencés pour supporter la ou les tiges d'immobilisation 23, et assurer l'immobilité de ces dernières relativement à la base 21. Parmi les passages 33, ceux qui sont associés aux tiges d'immobilisation 23 ont de préférence une forme sensiblement cylindrique (figures 3 et 4).

En outre, les passages 33 et les trous de visée 34 sont de préférence dimensionnés et espacés respectivement sur le bras 31 et sur le manchon 32 de telle manière que, quel que soit le déplacement du manchon 32, au moins une partie des passages 33 reste en regard des trous de visée 34 correspondants, dès lors que le déplacement du manchon 32 est inférieur à la course maximale admise correspondant sensiblement à la longueur des cavités 12 oblongues.

Selon une variante préférentielle de l'invention, le clou 5 d'arthrodèse se décline dans deux modes de réalisation préférentielle qui sont représentés sur la figure 5. Ainsi, dans le cas particulier de l'articulation de la cheville, le clou 5 peut se présenter sous la forme d'un premier clou 5A présentant une longueur d'environ 150 mm, ce premier clou 5A étant plus spécifiquement adapté à des tibias assez courts.

Au contraire, pour des tibias relativement longs, le clou 5 peut se décliner sous la forme d'un deuxième clou 5B présentant une longueur d'environ 180 mm.

Afin de permettre une mise en place du clou 5 quelle que soit sa longueur et sa déclinaison, l'instrument 20 et en particulier les moyens de visée 30 sont agencés de manière à permettre le montage de l'un ou l'autre des premier et deuxième clous 5A, 5B. En particulier, les bras 31 et les manchons 32 comportent respectivement des passages 33 et des trous de visée 34 spécifiquement adaptés à chacun des premier et deuxième clous 5A, 5B.

En outre, des repères peuvent être ménagés sur les manchons 32, et par exemple gravés sur la surface externe des manchons 32, de manière à indiquer, pour chacun des premier et deuxième clous 5A, 5B, quels trous de visée 34 doivent être utilisés.

Avantageusement, et tel que cela est représenté sur la figure 1, les moyens de visée 30 comportent un support de visée 37 conçu pour faciliter le repérage du positionnement des moyens de fixation 6 du clou 5, notamment les orifices de fixation 9, avec le premier os 2 (i.e. le calcanéum). Le support de visée 37 est en particulier destiné à permettre, à l'aide d'orifices 140, 141 aptes à recevoir des canons de perçage 143, le montage des vis de fixation 8 assurant le maintien du clou 5 relativement au premier os 2.

De façon préférentielle, le support de visée 37 est solidarisé avec le ou les manchons 32 par l'intermédiaire du moyen de maintien 40 formant ainsi, avec le ou les manchons 32, une platine de visée 200 mobile relativement à la platine de compression 210.

Avantageusement; le support de visée 37 se situe dans un plan sensiblement perpendiculaire au plan reliant les manchons 32 de manière à permettre la fixation de l'avant vers l'arrière des vis de fixation 8 dans le premier os.

De façon préférentielle, le support de visée 37, et/ou les manchons 32 et/ou le moyen de maintien 40 sont en matière plastique, formant ainsi de préférence une platine de visée 200 en matière plastique, avantageusement radiotransparente, afin de limiter les interférences avec les instruments de visualisation lors de l'intervention chirurgicale.

Ainsi, lors de la mise en place du clou 5 au sein de l'articulation, et de sa fixation avec le calcanéum, seule la platine de visée 200, séparable de la platine de compression 210, est utilisée, de telle sorte que la seule partie métallique visualisable par le chirurgien soit formée par le clou 5.

La platine de visée 200 et la platine de compression 210 forment ainsi avantageusement des ensembles monobloc amovibles l'un par rapport à l'autre.

Selon une variante non représentée, le support de visée 37 peut être formé par une pièce indépendante des autres moyens de visée 30, montée de façon amovible sur la base 21 par l'intermédiaire d'un ou plusieurs barreaux.

L'appareillage 1 d'arthrodèse selon l'invention permet donc d'assurer un montage facilité du clou 5 d'arthrodèse au sein de l'articulation, tout en permettant un rapprochement efficace et contrôlé des os formant l'articulation.

Un autre avantage de l'appareillage 1 d'arthrodèse selon l'invention est qu'il permet, grâce au montage amovible des moyens de visée 30, une mise en place méthodique du clou 5 d'arthrodèse.

Un autre avantage de l'appareillage 1 d'arthrodèse conforme à l'invention est qu'il permet, grâce aux nombreux moyens de repérage utilisés, d'éviter les erreurs de manipulation de la part du chirurgien.

Un autre avantage de l'appareillage 1 d'arthrodèse conforme à l'invention est qu'il présente un caractère entièrement démontable, ce qui facilite son nettoyage.

L'invention peut être utilisée selon une méthode chirurgicale pour réaliser une arthrodèse de l'articulation de la cheville, dans laquelle on utilise un appareillage 1 d'arthrodèse, comportant un clou 5 d'arthrodèse ainsi qu'un moyen de compression 22, 23, ladite méthode comportant les étapes suivantes :
- une étape de solidarisation du clou 5 avec le calcanéum,
- une étape de compression, au cours de laquelle d'une part on rapproche le calcanéum du tibia, en déplaçant le clou 5 relativement au tibia à l'aide du moyen de compression 22, 23 et d'autre part on contrôle l'amplitude de déplacement du clou 5 à l'aide de moyens de guidage 11 ménagés sur ledit clou 5.

Avantageusement, l'étape de compression est de préférence réalisée en utilisant une tige d'immobilisation 23 que l'on solidarise avec une partie fixe de l'instrument 20 et que l'on introduit, d'une part dans le tibia de manière à l'immobiliser, et d'autre part dans un moyen de guidage 11, de préférence une cavité 12 oblongue ménagée le long du clou 5, de telle sorte que lors de la compression, le clou 5 et la cavité 12 associée puissent se déplacer relativement à ladite tige d'immobilisation 23 sur une distance maximale correspondant à la longueur de la cavité 12 oblongue.

Ainsi, la cavité 12, de par sa forme oblongue, constitue un moyen de guidage et de contrôle du déplacement du clou 5 et donc de l'intensité de la compression exercée sur l'articulation. En outre, en raison de la forme oblongue de la cavité 12, le déplacement du clou 5 est nécessairement une translation, ce qui permet d'éviter une éventuelle déflexion du clou 5 susceptible de se produire lors de l'étape de compression.

Par ailleurs, le positionnement de la tige d'immobilisation 23 en regard du clou 5 lors de l'étape de compression confère à l'appareillage 1 une bonne rigidité d'ensemble, et permet d'effectuer une compression selon un axe sensiblement parallèle à l'axe d'extension du tibia.

Le fonctionnement de l'appareillage 1 d'arthrodèse et la méthode de mise en place du clou 5 vont maintenant être décrits dans le cas particulier de l'arthrodèse de la cheville.

Avant de procéder à l'arthrodèse, le chirurgien effectue tout d'abord une radiographie lui permettant de visualiser l'articulation, et de choisir l'appareillage d'arthrodèse, c'est-à-dire le clou 5 et l'instrument 20 adaptés à l'anatomie de l'articulation.

En particulier, dans le cas d'une arthrodèse de cheville, le chirurgien pourra choisir, suivant le cas, l'un des premier et deuxième clous 5A, 5B. Dans tous les cas, l'instrument 20 utilisé sera le même et il suffira au chirurgien de sélectionner, à l'aide des moyens de repérage réalisés à cet effet sur les moyens de visée 30, les trous de visée 34 susceptibles d'être utilisés avec le clou sélectionné. Lorsque le choix du clou adapté est effectué, le chirurgien peut positionner le clou 5 sur le moyen de liaison 24 à l'aide des moyens de repérage 25, 26. Grâce à l'unicité de la position du clou 5 sur le moyen de liaison 24, le support de visée 37, et en particulier les orifices 140, 141 se trouvent automatiquement en vis-à-vis des orifices de fixation 9 du clou 5 (figure 1).

Tel que cela est bien connu de l'homme du métier, le chirurgien doit également réaliser un logement pour le clou 5 au sein de l'articulation, ce logement traversant d'une part le calcanéum et d'autre part une partie du tibia et éventuellement l'astragale.

Le logement est de préférence réalisé de telle manière que lorsque le clou est introduit à l'intérieur de l'articulation, une poche 50 située vers l'extrémité interne du logement ménagé à l'intérieur du tibia reste non occupée par le clou 5 (figures 3 et 4).

Lorsque le clou 5 est introduit dans le logement, le chirurgien procède à sa fixation relativement au calcanéum à l'aide du support de visée 37.

Le chirurgien peut alors procéder au montage des vis de fixation 8 (figure 1) dans les orifices de fixation 9, en utilisant les canons de perçage 143 comme guides.

Une telle fixation est bien connue de l'homme du métier et il n'est pas nécessaire de la décrire plus en détails.

Lors de cette première étape de fixation du clou 5 avec le calcanéum, la platine de compression 210, métallique, n'est préférentiellement pas utilisée, de manière à éviter la perturbation des instruments (amplificateur etc...) par des parties métalliques autres que le clou 5.

La platine de visée 200 étant avantageusement en matière plastique, elle n'est pas « *vue »* par les instruments de telle sorte que le chirurgien peut visualiser plus précisément le clou 5 au cours de l'intervention.

Lorsque le clou 5 est fixé de façon solidaire avec le calcanéum, le chirurgien peut procéder à l'étape de compression, c'est-à-dire l'étape consistant à rapprocher le calcanéum du tibia.

Le chirurgien assure alors le montage de la platine de compression 210 sur la platine de visée 200, en emmanchant le ou les bras 31 dans le ou les manchons 32 correspondant(s), et en montant la base 21 sur le moyen de liaison 24, avec une possibilité de coulissement relatif.

L'étape de compression est illustrée sur les figures 3 et 4. La figure 3 montre en effet l'appareillage 1 d'arthrodèse dans une position précédant la compression où l'on voit apparaître un jeu J non nul entre les os 2, 3, 4 de l'articulation. Au contraire, la figure 4 illustre l'appareillage 1 d'arthrodèse dans une position après la mise en compression, dans laquelle le jeu J est sensiblement nul.

Pour procéder à la mise en compression, le chirurgien doit tout d'abord introduire les tiges d'immobilisation 23 du tibia d'une part à travers les passages 33 des bras 31 et d'autre part à travers les cavités 12 oblongues du clou 5. Les tiges d'immobilisation 23 traversent donc le tibia 3 de manière à l'immobiliser d'une part relativement au bras 31 et d'autre part relativement à la base 21.

Lorsque les tiges d'immobilisation 23 sont introduites, le chirurgien peut actionner le moyen de commande 22 notamment le volant 80, de manière à générer un déplacement discret ou continu du moyen de liaison 24 et donc du clou 5 associé relativement à la base 21 (ou platine de compression 210), fixe. Le déplacement maximal autorisé du clou 5 est pré-déterminé par la longueur des cavités 12 oblongues. Ce déplacement maximal autorisé est de préférence de l'ordre de 12 mm.

En interdisant un déplacement plus important du clou 5 d'arthrodèse, on évite que le chirurgien n'effectue une compression trop importante qui aurait pour effet de détériorer encore davantage l'articulation. La forme particulière des cavités 12 oblongues constitue donc un premier moyen de contrôle du taux de compression exercé sur l'articulation.

En se déplaçant, le clou 5 entraîne avec lui le calcanéum, et le rapproche progressivement du tibia tout en réduisant le volume occupé par la poche 50.

Les tiges d'immobilisation 23 qui, avant la mise en compression, se situent dans la partie supérieure des cavités oblongues 12, sont positionnées, après la mise en compression, dans la partie centrale ou inférieure desdites cavités 12 (figure 4).

On voit ainsi que les extrémités 12A, 12B des cavités 12 constituent des butées contre lesquelles les tiges d'immobilisation 23 viennent en appui lors du déplacement du clou 5.

Le déplacement du clou 5 est de préférence continu mais pourrait être discontinu et s'effectuer par paliers, notamment à l'aide d'un système à cliquets (non représenté).

Lorsque la mise en compression est terminée, le chirurgien peut procéder à la fixation du clou 5 relativement au tibia, et éventuellement à l'astragale. Une telle étape de fixation est représentée sur la figure 4.

Lorsque le clou 5 est solidarisé avec tous les os 2, 3, 4 formant l'articulation, la platine de visée 200, peut être intégralement démontée de la base 21 (ou de la platine de compression 210), facilitant ainsi le nettoyage et la stérilisation de l'appareil en vue d'une future utilisation.

### POSSIBILITE D'APPLICATION INDUSTRIELLE

L'invention trouve son application industrielle dans la réalisation d'appareillages et de clous d'arthrodèse.

## Revendications

1. Appareillage d'arthrodèse pour une articulation formée par au moins un premier os (2) et un deuxième os (3), du genre articulation de la cheville, ledit appareillage comportant :
- un clou (5) d'arthrodèse, pourvu de moyens de fixation (6) avec les os (2, 3, 4) formant l'articulation,
- un instrument (20) de mise en place dudit clou (5) d'arthrodèse au sein de l'articulation, comportant une base (21) ainsi qu'un moyen de compression (22, 23), destiné à permettre le rapprochement des premier et deuxième os formant l'articulation,
- un moyen de liaison (24), servant d'interface entre l'instrument (20) et le clou (5) d'arthrodèse,
**caractérisé en ce que** le moyen de compression comporte :
- un moyen de commande (22), agencé de manière à générer, par l'intermédiaire dudit moyen de liaison (24), un déplacement du clou d'arthrodèse relativement à ladite base (21) selon un axe longitudinal (X-X') sensiblement parallèle à l'axe d'extension dudit clou,
- un moyen d'immobilisation (23), agencé pour immobiliser le deuxième os (3) relativement à la base (21),
ledit clou comportant un moyen de guidage (11), ledit moyen de guidage (11) et ledit moyen d'immobilisation (23) étant agencés et conformés pour coopérer ensemble de manière à autoriser le déplacement du clou relativement au moyen d'immobilisation (23), et à permettre, lorsque le clou (5) est solidarisé avec le premier os (2), le rapprochement des premier os (2) et deuxième os (3).

2. Appareillage selon la revendication 1 **caractérisé en ce que** le moyen de guidage (11) est formé par au moins une cavité (12) oblongue, ménagée dans l'épaisseur du clou de manière à s'étendre selon un axe sécant de l'axe longitudinal (X-X'), ladite cavité (12) présentant une forme sensiblement allongée selon l'axe longitudinal (X-X').

3. Appareillage selon la revendication 2 **caractérisé en ce que** ladite cavité (12) oblongue est traversante.

4. Appareillage selon la revendication 2 ou 3 **caractérisé en ce que** le moyen de guidage (11) est formé par au moins deux cavités (12) oblongues ménagées l'une au dessus de l'autre le long dudit clou (5) et en alignement l'une de l'autre selon l'axe longitudinal (X-X').

5. Appareillage selon l'une quelconque des revendications 1 à 4 **caractérisé en ce que** les moyens de fixation (6) comportent deux trous de fixation (7), chacun destiné à coopérer avec au moins une vis de fixation (8) associée, de manière à solidariser le clou (5) avec le deuxième os (3).

6. Appareillage selon l'une quelconque des revendications 1 à 5 **caractérisé en ce que** les moyens de fixation (6) du clou (5) comportent au moins un orifice de fixation (9), destiné à coopérer avec au moins une vis de fixation (8) associée, de manière à solidariser le clou (5) avec le premier os (2).

7. Appareillage selon la revendication 6 **caractérisé en ce que** ledit au moins un orifice de fixation (9) s'étend en oblique relativement à l'axe longitudinal (X-X').

8. Appareillage selon la revendication 7 **caractérisé en ce que** l'angle (α) formé entre la direction d'extension (Y-Y') dudit au moins un orifice de fixation (9) oblique et l'axe longitudinal (X-X') est compris entre 95 degrés et 100 degrés.

9. Appareillage selon la revendication 7 ou 8 **caractérisé en ce que** les moyens de fixation (6) comportent deux orifices de fixation (9) du premier os.

10. Appareillage selon l'une des revendications 1 à 9 **caractérisé en ce que** les moyens de fixation (6) comportent une ouverture de fixation (10), agencée pour recevoir une vis de fixation (8) associée lorsque l'articulation comporte un troisième os situé entre le premier et le deuxième os, de manière à solidariser sensiblement le clou avec le troisième os.

11. Appareillage selon la revendication 10 **caractérisé en ce que** l'ouverture de fixation (10) est oblongue.

12. Appareillage selon l'une des revendications 2 à 11 **caractérisé en ce que** le moyen d'immobilisation du deuxième os est formé par au moins une tige d'immobilisation (23), conformée de manière à traverser, au moins en partie, d'une part le deuxième os pour le maintenir immobile relativement à la base (21) et d'autre part ladite au moins une cavité (12) oblongue.

13. Appareillage selon la revendication 12 **caractérisé en ce que** le moyen d'immobilisation est formé par deux tiges d'immobilisation (23).

14. Appareillage selon l'une quelconque des revendications 1 à 13 **caractérisé en ce que** l'instrument (20) comporte des moyens de visée (30), agencés de manière à faciliter le repérage du positionnement des moyens de fixation (6) sur le clou.

15. Appareillage selon la revendication 14 **caractérisé en ce que** les moyens de visée (30) comportent au moins un bras (31), monté sur ladite base (21) de manière à s'étendre sensiblement parallèlement à l'axe longitudinal (X-X').

16. Appareillage selon la revendication 15 **caractérisé en ce que** les moyens de visée comportent deux bras (31), disposés de part et d'autre du clou (5) monté sur l'instrument (20), en regard des cavités (12) oblongues.

17. Appareillage selon la revendication 15 ou 16 **caractérisé en ce que** les moyens de visée (30) comportent au moins un manchon (32) monté mobile sur au moins l'un des bras (31) avec une possibilité de coulissement suivant l'axe longitudinal (X-X').

18. Appareillage selon l'une des revendications 2 à 4, la revendication 12 et la revendication 17 **caractérisé en ce que** le bras (31) est pourvu de passages (33) tandis que le manchon (32) est pourvu de trous de visée (34), lesdits trous de visée (34) et les lesdits passages (33) étant agencés de telle manière que par déplacement du manchon (32), au moins une partie des trous de visée (34) puisse être mise en regard d'une partie desdits passages (33), formant ainsi des lumières de visée (35) situées chacune en vis-à-vis soit d'un moyen de fixation (6), soit d'une cavité (12) oblongue de manière à permettre le montage respectivement d'une vis de fixation (8) ou d'une tige d'immobilisation (23).

19. Appareillage selon la revendication 17 ou 18 **caractérisé en ce que** l'instrument (20) comporte un moyen de maintien (40), agencé pour solidariser le manchon (32) avec le clou (5) de telle sorte que le déplacement du clou (5) selon l'axe longitudinal (X-X') entraîne un déplacement sensiblement identique du manchon (32).

20. Appareillage selon l'une quelconque des revendications 1 à 19 **caractérisé en ce que** le moyen de liaison (24) et le clou (5) comportent des moyens de repérage (25, 26) agencés de manière à faciliter le positionnement du clou (5) relativement au moyen de liaison (24).

21. Appareillage selon la revendication 20 **caractérisé en ce que** les moyens de repérage (25, 26) sont formés par trois ergots (25) et trois fentes (26), agencés de telle manière que chaque fente (26) ne puisse être associée qu'avec un seul ergot (25) correspondant.

22. Appareillage selon l'une des revendications 14 à 19 **caractérisé en ce que** les moyens de visée (30) comportent un support de visée (37) conçu pour faciliter le repérage du positionnement des moyens de fixation (6) du clou (5) avec le premier os (2).

23. Appareillage selon les revendications 19 et 22 **caractérisé en ce que** la base (21) et ledit au moins un bras (31) forment une platine de compression (210) et **en ce que** ledit au moins un manchon (32) et le support de visée (37) forment une platine de visée (200), ladite platine de visée (200) étant montée mobile relativement à la platine de compression (210).

24. Appareillage selon la revendication 23 **caractérisé en ce que** la platine de compression (210) est montée de façon amovible relativement à la platine de visée (200).

25. Appareillage selon la revendication 24 **caractérisé en ce que** la platine de compression (210) est en métal, et **en ce que** la platine de visée (200) est en matière plastique.

26. Appareillage selon l'une des revendications 1 à 25 **caractérisé en ce que** le moyen de commande 22 est formé par un volant 80.

## Patentansprüche

1. Arthrodesegerät für ein Gelenk, das von mindestens einem ersten Knochen (2) und einem zweiten Knochen (3) gebildet ist, vom Typ Knöchelgelenk, wobei das Gerät umfasst:
- einen Arthrodesenagel (5), der mit Mitteln (6) zur Befestigung mit den Knochen (2, 3, 4), die das Gelenk bilden, versehen ist,
- ein instrument (20) zum Anbringen des Arthrodesenagels (5) im Gelenk, umfassend eine Basis (21) sowie ein Kompressionsmittel (22, 23), das dazu bestimmt ist, die Annäherung des ersten und zweiten Knochens, die das Gelenk bilden, zu ermöglichen,
- ein Verbindungsmittel (24), das als Schnittstelle zwischen dem Instrument (20) und dem Arthrodesenagel (5) dient,
**dadurch gekennzeichnet, dass** das Kompressionsmittel umfasst:
- ein Steuermittel (22), das derart angeordnet ist, das es mit Hilfe des Verbindungsmittels (24) eine Verschiebung des Arthrodesenagels in Bezug zur Basis (21) entlang einer Längsachse (X-X') im Wesentlichen parallel zur Ausdehnungsachse des Nagels erzeugt,
- ein Fixierungsmittel (23), das derart vorgesehen ist, dass es den zweiten Knochen (3) in Bezug zur Basis (21) fixiert,
wobei der Nagel ein Führungsmittel (11) umfasst, wobei das Führungsmittel (11) und das Fixierungsmittel (23) derart angeordnet und ausgebildet sind, dass sie zusammenwirken, um die Verschiebung des Nagels in Bezug zum Fixierungsmittel (23) zu gestatten, und, wenn der Nagel (5) mit dem ersten Knochen (2) verbunden ist, die Annäherung des ersten Knochens (2) und des zweiten Knochens (3) zu ermöglichen.

2. Gerät nach Anspruch 1, **dadurch gekennzeichnet, dass** das Führungsmittel (11) von mindestens einem länglichen Hohlraum (12) gebildet ist, der in der Dicke des Nagels ausgenommen ist, um sich entlang einer Schnittachse der Längsachse (X-X') zu erstrecken, wobei der Hohlraum (12) eine im Wesentlichen längliche Form entlang der Längsachse (X-X') aufweist.

3. Gerät nach Anspruch 1, **dadurch gekennzeichnet, dass** der längliche Hohlraum (12) durchgehend ist.

4. Gerät nach Anspruch 2 oder 3, **dadurch gekennzeichnet, dass** das Führungsmittel (11) von mindestens zwei länglichen Hohlräumen (12) gebildet ist, die übereinander entlang des Nagels (5) und in Ausrichtung zueinander entlang der Längsachse (X-X') ausgenommen sind.

5. Gerät nach einem der Ansprüche 1 bis 4, **dadurch gekennzeichnet, dass** die Befestigungsmittel (6) zwei Befestigungslöcher (7) umfassen, die jeweils dazu bestimmt sind, mit mindestens einer zugehörigen Befestigungsschraube (8) zusammenzuwirken, um den Nagel (5) mit dem zweiten Knochen (3) zu verbinden.

6. Gerät nach einem der Ansprüche 1 bis 5, **dadurch gekennzeichnet, dass** die Befestigungsmittel (6) des Nagels (5) mindestens eine Befestigungsöffnung (9) umfassen, die dazu bestimmt ist, mit mindestens einer zugehörigen Befestigungsschraube (8) zusammenzuwirken, um den Nagel (5) mit dem ersten Knochen (2) zu verbinden.

7. Gerät nach Anspruch 6, **dadurch gekennzeichnet, dass** sich die mindestens eine Befestigungsöffnung (9) schräg zur Längsachse (X-X') erstreckt.

8. Gerät nach Anspruch 7, **dadurch gekennzeichnet, dass** der Winkel (α), der zwischen der Ausdehnungsrichtung (Y-Y') der mindestens einen schrägen Befestigungsöffnung (9) und der Längsachse (X-X') gebildet ist, zwischen 95 Grad und 100 Grad beträgt.

9. Gerät nach Anspruch 7 oder 8, **dadurch gekennzeichnet, dass** die Befestigungsmittel (6) zwei Öffnungen (9) zur Befestigung des ersten Knochens umfassen.

10. Gerät nach einem der Ansprüche 1 bis 9, **dadurch gekennzeichnet, dass** die Befestigungsmittel (6) eine Befestigungsöffnung (10) umfassen, die derart vorgesehen ist, dass sie eine zugehörige Befestigungsschraube (8) aufnimmt, wenn das Gelenk einen dritten Knochen umfasst, der sich zwischen dem ersten und dem zweiten Knochen befindet, um den Nagel im Wesentlichen mit dem dritten Knochen zu verbinden.

11. Gerät nach Anspruch 10, **dadurch gekennzeichnet, dass** die Befestigungsöffnung (10) länglich ist.

12. Gerät nach einem der Ansprüche 2 bis 11, **dadurch gekennzeichnet, dass** das Fixierungsmittel des zweiten Knochens von mindestens einer Fixierungsstange (23) gebildet ist, die derart ausgebildet ist, dass sie zumindest teilweise einerseits durch den zweiten Knochen hindurchgeht, um ihn in Bezug zur Basis (21) unbeweglich zu halten, und andererseits durch den mindestens einen länglichen Hohlraum (12).

13. Gerät nach Anspruch 12, **dadurch gekennzeichnet, dass** das Fixierungsmittel von zwei Fixierungsstangen (23) gebildet ist.

14. Gerät nach einem de Ansprüche 1 bis 13, **dadurch gekennzeichnet, dass** das Instrument (20) Sichtmittel (30) umfasst, die derart angeordnet sind, dass sie die Ortung der Positionierung der Befestigungsmittel (6) auf dem Nagel erleichtem.

15. Gerät nach Anspruch 14, **dadurch gekennzeichnet, dass** die Sichtmittel (30) mindestens einen Arm (31) umfassen, der auf der Basis (21) derart montiert ist, dass er sich im Wesentlichen parallel zur Längsachse (X-X') erstreckt.

16. Gerät nach Anspruch 15, **dadurch gekennzeichnet, dass** die Sichtmittel zwei Arme (31) umfassen, die beiderseits des Nagels (5), der auf dem Instrument (20) montiert ist, gegenüber den länglichen Hohlräumen (12) angeordnet sind.

17. Gerät nach Anspruch 15 oder 16, **dadurch gekennzeichnet, dass** die Sichtmittel (30) mindestens eine Hülse (32) umfassen, die beweglich auf mindestens einem der Arme (31) mit einer Möglichkeit des Gleitens entlang der Längsachse (X-X') montiert ist.

18. Gerät nach einem der Ansprüche 2 bis 4, Anspruch 12 und Anspruch 17, **dadurch gekennzeichnet, dass** der Arm (31) mit Durchgängen (33) versehen ist, während die Hülse (32) mit Sichtlöchern (34) versehen ist, wobei die Sichtlöcher (34) und die Durchgänge (33) derart angeordnet sind, dass durch Verschieben der Hülse (32) zumindest ein Teil der Sichtlöcher (34) gegenüber einem Teil der Durchgänge (33) angeordnet werden kann, wodurch Sichtöffnungen (35) gebildet werden, die sich jeweils entweder gegenüber einem Befestigungsmittel (6) oder einem länglichen Hohlraum (12) befinden, um die Montage einer Befestigungsschraube (8) bzw. einer Fixierungsstange (23) zu ermöglichen.

19. Gerät nach Anspruch 17 oder 18, **dadurch gekennzeichnet, dass** das Instrument (20) ein Haltemittel (40) umfasst, das derart angeordnet ist, dass es die Hülse (32) mit dem Nagel (5) derart verbindet, dass die Verschiebung des Nagels (5) entlang der Längsachse (X-X') zu einer im Wesentlichen identischen Verschiebung der Hülse (32) führt.

20. Gerät nach einem der Ansprüche 1 bis 19, **dadurch gekennzeichnet dass** das Verbindungsmittel (24) und der, Nagel (5) Ortungsmittel (25, 26) umfassen, die derart angeordnet sind, dass sie die Positionierung des Nagels (5) in Bezug zum Verbindungsmittel (24) erleichtern.

21. Gerät nach Anspruch 20, **dadurch gekennzeichnet, dass** die Ortungsmittel (25, 26) von drei Haken (25) und drei Schlitzen (26) gebildet sind, die derart angeordnet sind, dass jeder Schlitz (26) nur mit einem entsprechenden Haken (25) verbunden werden kann.

22. Gerät nach einem der Ansprüche 14 bis 19, **dadurch gekennzeichnet, dass** die Sichtmittel (30) eine Sichtstütze (37) umfassen, die derart ausgeführt ist, dass sie die Ortung der Positionierung der Befestigungsmittel (6) des Nagels (5) mit dem ersten Knochen (2) erleichtern.

23. Gerät nach einem der Ansprüche 19 und 22, **dadurch gekennzeichnet, dass** die Basis (21) und der mindestens eine Arm (31) eine Kompressionsplatte (210) bilden, und dass die mindestens eine Hülse (32) und die Sichtstütze (37) eine Sichtplatte (200) bilden, wobei die Sichtplatte (200) beweglich in Bezug zur Kompressionsplatte (210) montiert ist.

24. Gerät nach Anspruch 23, **dadurch gekennzeichnet, dass** die Kompressionsplatte (210) in Bezug zu der Sichtplatte (200) abnehmbar montiert ist.

25. Gerät nach Anspruch 24, **dadurch gekennzeichnet, dass** die Kompressionsplatte (210) aus Metall ist, und dass die Sichtplatte (200) aus Kunststoff ist.

26. Gerät nach einem der Ansprüche 1 bis 25, **dadurch gekennzeichnet, dass** das Steuermittel (22) von einem Steuerrad (80) gebildet ist.

## Claims

1. An arthrodesis apparatus for a joint formed by at least a first bone (2) and a second bone (3), such as the ankle joint, said apparatus comprising:
- an arthrodesis nail (5) provided with fastener means (6) for fastening to the bones (2, 3, 4) constituting the joint;
- an instrument (20) for putting said arthrodesis nail (5) into place within the joint, the instrument comprising a base (21) together with compression means (22, 23) designed to enable the first and second bones forming the joint to be moved towards each other; and
- link means (24) serving as an interface between the instrument (20) and the arthrodesis nail (5);
the apparatus being **characterized in that** the compression means comprise:
- control means (22) arranged in such a manner as to act via said link means (24) to move the arthrodesis nail relative to said base (21) along a longitudinal axis (X-X') substantially parallel to the axis along which said nail extends; and
- holder means (23) arranged to prevent the second bone (3) from moving relative to the base (21);
said nail including guide means (11), said guide means (11) and said holder means (23) being arranged and shaped to co-operate together in such a manner as to allow the nail to move relative to the holder means (23) and to enable the first bone (2) and the second bone (3) to be moved towards each other once the nail (5) has been secured to the first bone (2).

2. Apparatus according to claim 1, **characterized in that** the guide means (11) are formed by at least one oblong cavity (12) provided in the thickness of the nail so as to extend along the axis that intersects the longitudinal axis (X-X'), said cavity (12) presenting a shape that is substantially elongate along the longitudinal axis (X-X').

3. Apparatus according to claim 2, **characterized in that** said oblong cavity (12) is a through cavity.

4. Apparatus according to claim 2 or claim 3, **characterized in that** the guide means (11) are formed by at least two oblong cavities (12) formed one above the other along said nail (5) and in alignment with each other along the longitudinal axis (X-X').

5. Apparatus according to any one of claims 1 to 4, **characterized in that** the fastener means (6) comprise two fastener holes (7) each designed to co-operate with at least one associated fastener screw (8) so as to secure the nail (5) to the second bone (3).

6. Apparatus according to any one of claims 1 to 5, **characterized in that** the fastener means (6) of the nail (5) include at least one fastener orifice (9) designed to co-operate with at least one associated fastener screw (8) so as to secure the nail (5) to the first bone (2).

7. Apparatus according to claim 6, **characterized in that** said at least one fastener orifice (9) extends obliquely relative to the longitudinal axis (X-X').

8. Apparatus according to claim 7, **characterized in that** the angle (α) formed between the direction (Y-Y') in which said at least one oblique fastener orifice (9) extends and the longitudinal axis (X-X') lies in the range 95° to 100°.

9. Apparatus according to claim 7 or claim 8, **characterized in that** the fastener means (6) include at least two fastener orifices (9) for fastening the first bone.

10. Apparatus according to any one of claims 1 to 9, **characterized in that** the fastener means (6) include at least one fastener opening (10) arranged to receive an associated fastener screw (8) when the joint includes a third bone situated between the first and second bone, so as to secure the nail substantially with the third bone.

11. Apparatus according to claim 10, **characterized in that** the fastener opening (10) is oblong.

12. Apparatus according to any one of claims 2 to 11, **characterized in that** the holder means for the second bone is formed by at least one holder rod (23) shaped so as to pass at least in part, both through the second bone so as to hold it stationary relative to the base (21), and through said at least one oblong cavity (12).

13. Apparatus according to claim 12, **characterized in that** the holder means is formed by two holder rods (23).

14. Apparatus according to any one of claims 1 to 13, **characterized in that** the instrument (20) includes aiming means (30) arranged to facilitate identifying the positioning of the fastener means (6) on the nail.

15. Apparatus according to claim 14, **characterized in that** the aiming means (30) comprise at least one arm (31) mounted on said base (21) in such a manner as to extend substantially parallel to the longitudinal axis (X-X').

16. Apparatus according to claim 15, **characterized in that** the aiming means comprise two arms (31) disposed on either side of the nail (5) mounted on the instrument (20), facing the oblong cavities (12).

17. Apparatus according to claim 15 or claim 16, **characterized in that** the aiming means (30) include at least one sleeve (32) mounted to move on at least one of the arms (31) with the possibility of sliding along the longitudinal axis (X-X').

18. Apparatus according to any one of claims 2 to 4, claim 12, and claim 17, **characterized in that** the arm (31) is provided with passages (33) while the sleeve (32) is provided with aiming holes (34), said aiming holes (34) and said passage (33) being arranged in such a manner that by moving the sleeve (32) at least some of the aiming holes (34) can be caused to face some of said passages (33), thus forming aiming windows (35), each situated in register either with fastener means (6) or with an oblong cavity (12) so as to enable a fastener screw (8) or a holder rod (23) respectively to be mounted.

19. Apparatus according to claim 17 or claim 18, **characterized in that** the instrument (20) includes spacer means (40) arranged to secure the sleeve (32) to the nail (5) in such a manner that moving the nail (5) along the longitudinal axis (X-X') causes substantially identical movement of the sleeve (32).

20. Apparatus according to any one of claims 1 to 19, **characterized in that** the link means (24) and the nail (5) include marker means (25, 26) arranged in such a manner as to facilitate positioning the nail (5) relative to the link means (24).

21. Apparatus according to claim 20, **characterized in that** the marker means (25, 26) are formed by three studs (25) and by three slots (26) arranged in such a manner that each slot (26) can be associated with only one corresponding stud (25).

22. Apparatus according to any one of claims 14 to 19, **characterized in that** the aiming means (30) comprise an aiming support (37) designed to facilitate identifying the positioning of the fastener means (6) for fastening the nail (5) to the first bone (2).

23. Apparatus according to claims 19 and 22, **characterized in that** the base (21) and said at least one arm (31) form a compression structure (210) and **in that** said at least one sleeve (32) and the aiming support (37) forming an aiming structure (200), said aiming structure (200) being mounted to move relative to the compression structure (210).

24. Apparatus according to claim 23, **characterized in that** the compression structure (210) is removably mounted relative to the aiming structure (200).

25. Apparatus according to claim 24, **characterized in that** the compression structure (210) is made of metal, and **in that** the aiming structure (200) is made of plastics material.

26. Apparatus according to any one of claims 1 to 25, **characterized in that** the control means (22) are formed by a handwheel (80).
